# EUROPÄISCHE PATENTSCHRIFT

(11) **EP 3 589 232 B1**
(45) Veröffentlichungstag und Bekanntmachung des Hinweises auf die Patenterteilung: **06.01.2021**
(21) Anmeldenummer: 18711839.3
(22) Anmeldetag: 02.03.2018
(51) Int. Cl.: A61C 13/00

(54) **VERFAHREN ZUR KONSTRUKTION EINER RESTAURATION**
METHOD FOR CONSTRUCTING A RESTORATION
PROCÉDÉ DE RÉALISATION D'UNE RESTAURATION

(30) Priorität: 03.03.2017 DE 102017203475
(43) Veröffentlichungstag der Anmeldung: 08.01.2020
(73) Patentinhaber: SIRONA Dental Systems GmbH, 64625 Bensheim (DE)
(72) Erfinder: WEY, Peter, 5436 Würenlos (CH)
(74) Vertreter: Özer, Alpdeniz
(86) Internationale Anmeldenummer: PCT/EP2018/055145
(87) Internationale Veröffentlichungsnummer: WO 2018/158411

(56) Entgegenhaltungen:
- WO-A1-98/15234
- WO-A1-2018/022752
- US-A1- 2012 239 364
- RAITH STEFAN ET AL: "Artificial Neural Networks as a powerful numerical tool to classify specific features of a tooth based on 3D scan data", COMPUTERS IN BIOLOGY AND MEDICINE, NEW YORK, NY, US, vol. 80, 27 November 2016 (2016-11-27), pages 65-76, XP029910822, ISSN: 0010-4825, DOI: 10.1016/J.COMPBIOMED.2016.11.013

## Beschreibung

Die Erfindung betrifft ein Verfahren zur Konstruktion einer Restauration, wobei mittels einer dentalen Kamera eine Zahnsituation vermessen wird und ein 3D-Modell der Zahnsituation erzeugt wird.

### Stand der Technik

Aus dem Stand der Technik sind mehrere Verfahren zur Planung von Restaurationen bekannt, wobei der Benutzer manuell den Restaurationstyp bestimmen muss und die Zahnnummer der herzustellenden Restauration angeben muss.

Ein Nachteil dieses Verfahrens besteht darin, dass der Benutzer einen falschen Zahntyp oder eine falsche Zahnnummer der herzustellenden Restauration auswählen kann, so dass es zu fehlerhaften Restaurationen kommen kann.

US 2012/0239364 A1 offenbart ein Verfahren zur automatisierten Planung eines Zahnimplantats, wobei ein 3D-Modell der Zahlensituation analysiert wird und Zonen im Kiefer zur Einsetzung von möglichen Implantaten ermittelt werden. Anschließend werden die Dimensionen, die Position und Orientierung der Implantate bestimmt. Eine Zahnnummer kann beispielsweise auf der Grundlage einer Zahnbreite bestimmt werden.

WO 98/15234 A1 offenbart ein Verfahren zur Diagnose einer Zahnsituation, wobei ein Zahnarzt oder ein Computer zu einer entsprechenden Präparation ein passendes Inlay, Onlay oder Krone auswählt.

WO 2018/022752 A1 offenbart ein Verfahren zum Erkennen zahnmedizinische Informationen eines Zahnmodells, wobei ein neuronales Netzwerk trainiert wird, wobei das trainierten neuronale Netzwerk automatisch auf der Grundlage des Zahnmodells ein Modell einer passenden Restauration, wie einer Krone, erzeugen kann.

Die Aufgabe der vorliegenden Erfindung besteht daher darin, ein Verfahren bereitzustellen, das den Zahntyp und/oder die Zahnnummer der herzustellenden Restauration automatisch bestimmen kann.

### Darstellung der Erfindung

Die Erfindung betrifft ein Verfahren gemäß Anspruch 1, zur Konstruktion einer Restauration, wobei mittels einer dentalen Kamera eine Zahnsituation vermessen wird und ein 3D-Modell der Zahnsituation erzeugt wird. Dabei wird ein computergestützter Erkennungsalgorithmus auf das 3D-Modell der Zahnsituation angewendet, wobei ein Restaurationstyp automatisch ermittelt wird.

Die Restauration kann eine beliebige Restauration sein, die beispielsweise mittels eines CAD/CAM-Verfahrens herstellbar ist. Die dentale Kamera kann eine beliebige dreidimensionale dentale Kamera sein, die beispielsweise auf einem Streifenprojektionsverfahren oder einem konfokalen Vermessungsverfahren beruht. Die Zahnsituation kann die unmittelbare Umgebung der einzusetzenden Restauration oder auch einen größeren Bereich um die einzusetzende Restauration umfassen. Die Vermessung mittels der dentalen Kamera kann aus unterschiedlichen Richtungen, wie einer okklusalen Richtung, einer lingualen Richtung, einer bukkalen Richtung oder einer labialen Richtung, erfolgen. Nach der Vermessung mittels der dentalen Kamera wird das 3D-Modell der Zahnsituation erzeugt. Anschließend wird der Erkennungsalgorithmus auf das 3D-Modell der Zahnsituation angewendet. Der Erkennungsalgorithmus kann beispielsweise auf einem künstlichen neuronalen Netz für maschinelles Lernen oder auf einem Template-matching-Verfahren beruhen. Nach der Analyse des 3D-Modells des Erkennungsalgorithmus werden der Restaurationstyp und/oder die Zahnnummer des mindestens einen Zahns für die einzusetzende Restauration automatisch bestimmt.

Ein Vorteil dieses Verfahrens besteht darin, dass die Ermittlung des Restaurationstyps und der Zahnnummer automatisch ohne eine Benutzerinteraktion erfolgt. Dadurch werden also mögliche Bedienfehler durch den Benutzer verhindert und die Dauer der Planung einer Restauration verkürzt.

Vorteilhafterweise kann der Restaurationstyp ein Inlay, eine Krone, eine Brücke, ein Abutment, ein Pontic oder ein Veneer sein.

Die Brücke kann beispielsweise unter Verwendung von Implantaten und Abutments am Kieferknochen befestigt werden oder an den Zahnstümpfen der benachbarten gesunden Zähne befestigt werden. Die Brücke kann festsitzend oder abnehmbar sein. Die Brücke kann auch eine Basis-Brücke sein, die aus einer Basis aus einer Metalllegierung und einem Aufbau aus Keramik oder einem Kunststoff besteht.

Ein Abutment ist ein Stützpfeiler, der als Verbindungsteil zwischen einem Zahnimplantat und einer Restauration, wie einer Zahnkrone, dient. Ein Abutment kann lösbar oder fest am Implantat befestigt werden. Implantat-Abutments können nach der Herstellungsform eingeteilt werden. Dabei wird zwischen konfektionierten, angussfähigen bzw. überpressbaren und CAD/CAM-Implantat-Abutments unterschieden. Konfektionierte Abutments werden in verschiedenen Größen, Formen und Abwinklungen sowie als beschleifbare oder nicht beschleifbare Varianten angeboten. Einteilige Implantate haben Abutments integriert. Die mittels eines CAD/CAM-Verfahrens angefertigten Abutments können sowohl in der Achsenneigung als auch in der Formgestaltung an die gegebene Zahnsituation individuell angepasst werden. Zahnfarbige Abutments finden bei ästhetischen Restaurationen, insbesondere im Frontzahnbereich Anwendung, so dass der optische Eindruck eines natürlichen Zahns möglichst nachgeahmt werden soll. Abutments werden üblicherweise aus Titan oder einer Keramik hergestellt.

Ein Veneer ist eine Verblendschale aus einer dünnen, lichtdurchlässigen Keramikschale, insbesondere für die Frontzähne.

Ein Inlay ist eine Einlagefüllung, die in einer Präparation eines Zahns eingesetzt wird. Im Gegensatz zu plastischem Füllungsmaterial, das in weicher Konsistenz mittels Formhilfen in den Zahn eingebracht wird und anschließend aushärtet ist das Inlay ein passgenaues Werkstück, das in die Präparation des Zahns eingeklebt wird.

Ein Pontic ist eine Brücke, die auf Zahnstümpfe von gesunden benachbarten Zähnen oder auf Implantat-Abutments aufgeklebt wird.

Das 3D-Modell der Zahnsituation wird also mittels des Erkennungsalgorithmus analysiert, um eines der genannten Restaurationstypen zu bestimmen.

Die Zahnsituation weist mindestens eine Präparation oder eine implantatgetragene Mesostruktur, wie ein Abutment, zum Einsetzen der herzustellenden Restauration auf.

Eine Mesostruktur, wie eine Abutment, dient als Verbindungslied zwischen einem Implantat und einer Restauration, wie beispielsweise einer Zahnkrone. Anhand der Form der Präparation kann eine passende Restauration, wie ein Inlay, eine Krone, ein Pontic oder ein Veneer, konstruiert werden. Die herzustellende Restauration wird also passend in die Präparation eingeklebt. Das 3D-Modell der Zahnsituation kann also eine Präparation oder ein Abutment umfassen.

Der computergestützte Erkennungsalgorithmus weist ein künstliches neuronales Netz für maschinelles Lernen (engl. Convolutional Neural Network; CNN) auf, wobei anhand des 3D-Modells der Zahnsituation die Form der Präparation oder der implantatgetragenen Mesostruktur analysiert wird und ein passender Restaurationstyp ausgewählt wird.

Ein künstliches neuronales Netzwerk für maschinelles Lernen (CNN) ist ein Computeralgorithmus, der die automatische Erkennung des Restaurationstyps ermöglicht. Im Folgenden wird ein Verfahren unter Verwendung eines CNN erläutert.

Ein Convolutional Neural Network (CNN), zu Deutsch "faltendes neuronales Netzwerk", ist ein feedforward künstliches neuronales Netz. Es handelt sich um ein von biologischen Prozessen inspiriertes Konzept im Bereich des maschinellen Lernens. Convolutional Neural Networks finden Anwendung in zahlreichen, modernen Technologien der künstlichen Intelligenz, vornehmlich bei der maschinellen Verarbeitung von Bild- oder Audiodaten.

Grundsätzlich besteht die Struktur eines klassischen CNN aus einem Convolutional Layer, gefolgt von einem Pooling Layer. Diese Einheit kann sich prinzipiell beliebig oft wiederholen, bei ausreichend Wiederholungen spricht man dann von Deep Convolutional Neural Networks, die in den Bereich Deep Learning fallen.

Die CNN lernen dadurch, dass freie Parameter bzw. Klassifikatoren der Convolution-Kernel pro Layer und deren Gewichtung bei der Verrechnung zum nächsten Layer gelernt werden. Im ersten Schritt wird das 3D-Modell der Zahnsituation mittels der dentalen Kamera aufgenommen. Dabei kann die Aufnahme aus einer okklusalen Richtung, einer lingualen Richtung und/oder einer labialen Richtung erfolgen. Im zweiten Schritt wird die Position der einzusetzenden Restauration bestimmt. Dies kann beispielsweise manuell durch den Benutzer erfolgen, indem der Benutzer die Position der einzusetzenden Restauration in einer graphischen Darstellung des 3D-Modells der Zahnsituation auswählt. Die Bestimmung der Position der einzusetzenden Restauration kann auch automatisch erfolgen, indem die Aufnahmerichtung und das Zentrum des Aufnahmebereichs ermittelt werden. Die Position der einzusetzenden Restauration entspricht dann einer Position auf die die dentale Kamera während der Vermessung zeigt.

In einem weiteren Schritt wird in einem Bereich der einzusetzenden Restauration, der eine Präparation umfassen kann, eine Analyse durchgeführt, wobei das 3D-Modell der Zahnsituation aus unterschiedlichen Richtungen, wie einer okklusalen Richtung, einer mesialen Richtung, einer lingualen Richtung, bukkalen Richtung und/oder einer labialen Richtung, in mehrere Schichten, sogenannte Höhenfelder, zerlegt werden.

Die Höhenfelder des 3D-Modells können alternativ zur Bildung von mehreren Schichten des 3D-Modells auch dadurch gebildet werden, dass die Helligkeit jedes Pixels eines Höhenfeldes dem Abstand zwischen einem jeweiligen Oberflächenpunkt des 3D-Modells relativ zu einer festgelegten Position einer virtuellen Kamera entspricht. Ein solches Höhenfeld aus einer okklusalen Richtung würde dann beispielsweise dunkle Bereiche enthalten, die Oberflächenbereichen des 3D-Modells entsprechen, die weiter weg von der Kamera angeordnet sind, und helle Bereiche enthalten, die Oberflächenbereichen des 3D-Modells entsprechen, die näher an der Kamera angeordnet sind.

Die Höhenfelder des 3D-Modells werden als Eingabe eines Maschinen-Learning-Systems verwendet, welches unter Verwendung einer Sammlung einer Vielzahl von 3D-Modellen unterschiedlicher Zahnsituationen trainiert wurde.

In einem weiteren Schritt wird das 3D-Modell der Zahnsituation mittels des Maschinen-Learning-Systems analysiert und als Ausgabe wird ein passender Restaurationstyp vorgeschlagen und/oder eine Zahnnummer der einzusetzenden Restauration vorgeschlagen.

Im weiteren Verfahren kann anhand des bekannten 3D-Modells der Zahnsituation inklusive der Form der Präparation anhand des ermittelten Restaurationstyps und anhand der ermittelten Zahnnummer der einzusetzenden Restaurationen ein 3D-Modell der einzusetzenden Restauration berechnet werden. Dabei kann ein Präparationsrand automatisch ermittelt werden und Strukturen wie benachbarte Zähne, Gegenzähne und die Form der Präparation berücksichtigt werden. Die Konstruktion der Restauration kann dabei vollautomatisch erfolgen.

Im nächsten Schritt kann unter Verwendung des konstruierten 3D-Modells der Restauration die Restauration vollautomatisch mittels einer CAM-Schleifmaschine aus einem Rohling hergestellt werden. Das erläuterte Verfahren hat also den Vorteil, dass die Restauration vollautomatisch ohne eine Benutzerinteraktion hergestellt werden kann.

Das Maschinen-Learning-System kann aus einem oder mehreren CNN-Netzwerken bestehen.

Als Eingabe für das CNN-Netzwerk können auch Farbinformationen der Zahnsituation verwendet werden. Die Farbinformationen werden dann den Oberflächenpunkten des 3D-Modells der Zahnsituation zugeordnet.

Im Folgenden wird das Verfahren zum Training bzw. Parametrisierung des Maschinen-Learning-Systems bestehend aus einem oder mehreren CNN-Netzwerken erläutert. Im ersten Schritt wird eine große Anzahl bekannter 3D-Modelle von Zahnsituationen mit einem bekannten Restaurationstyp und einer bekannten Zahnnummer analysiert. Dabei werden mögliche Eingabedaten bzw. Inputdaten generiert. Die Inputdaten werden so erzeugt, dass alle möglichen Freiheitsgrade in den Inputdaten vorhanden sind. Dies wird unter Verwendung einer Data-Augmentation erreicht. Dazu werden die 3D-Modelle der Zahnsituationen um die festgelegten Freiheitsgrade rotiert und/oder entlang der Freiheitsgrade skaliert. Die einzelnen CNN-Netzwerke werden dann auf die einzelnen 3D-Daten der einzelnen 3D-Modelle der Zahnsituationen angewendet, um die CNN-Netzwerke zu trainieren.

Bei diesem Verfahren lernen die CNN-Netzwerke also automatisch anhand eines Trainingssets mehrere 3D-Modelle von Zahnsituationen mit bereits bekanntem Restaurationstyp und Zahnnummer.

Alternativ zur Verwendung von CNN-Netzwerken kann auch ein nicht erfindungsgemäßes alternatives Verfahren aus den Maschine-Learning/Deep-Learning-Bereich auf der Basis von Deep-Belief-Networks verwendet werden, bei dem Höhenfelder ebenfalls als Inputdaten verwendet werden.

Bei einer nicht erfindungsgemäßen. Alternative könnte auch ein hybrides Verfahren zur Analyse des 3D-Modells der Zahnsituation verwendet werden, wobei durch einen Benutzer manuell definierte Klassifikatoren festgelegt werden, wobei die Parameter der festgelegten Klassifikatoren mittels des Trainingssets einer Vielzahl bekannter 3D-Modelle mehrerer Zahnsituationen trainiert werden.

Der Vorteil eines CNN-Netzwerks besteht jedoch darin, dass die Parameterwerte der internen Convolution-Filter und die Weiterverarbeitung der Filterausgaben bei der Analyse des Trainingssets mitgelernt werden und deshalb keine weitere Benutzerspezifikation notwendig ist.

Die Klassifikatoren bzw. Merkmale werden also automatisch festgelegt und bei der Analyse des Trainingssets verfeinert. Die automatisch ermittelten Klassifikatoren eines 3D-Modells einer Zahnsituation könnten beispielsweise eine Gesamtfläche einer Präparation oder der Verlauf des Präparationsrandes sein.

Das CNN-Netzwerk kann beispielsweise aus mehreren Layern bestehen, wobei in einem ersten Layer einfache Klassifikatoren, wie Kanten, flache Oberflächen oder Bereiche gleicher Helligkeit, automatisch identifiziert werden. In einem zweiten Layer werden die Klassifikatoren automatisch verfeinert. Die Klassifikatoren im zweiten Layer können beispielsweise die relative Anordnung der Kanten zueinander, die relative Richtung der Kanten oder der Verlauf der Kanten sein. In den weiteren Layern werden die Klassifikatoren immer weiter verfeinert und werden dadurch immer komplexer. Auf diese Art und Weise lernt das CNN- Netzwerk selbstständig anhand des 3D-Modells als Eingabeparameter den passenden Restaurationstyp und/oder die Zahnnummer als Ausgabeparameter automatisch zu bestimmen.

Vorteilhafterweise kann zusätzlich anhand einer Oberfläche mindestens eines Restzahns des jeweiligen Zahns für die einzusetzende Restauration und/oder der Nachbarzähne relativ zu dem jeweiligen Zahn die Zahnnummer und/oder eine Position des Zahns bestimmt werden.

Die Zahnnummer bzw. die Position des Zahns wird also anhand der Oberfläche des Restzahns und/oder der Nachbarzähne automatisch ermittelt. Denn der Erkennungsalgorithmus erkennt automatisch anhand der Form, der Abmessungen und der Ausrichtung der Nachbarzähne um welche Zahnnummer der herzustellenden Restauration es sich handelt.

Vorteilhafterweise kann der computergestützte Erkennungsalgorithmus ein Template-matching-Verfahren mit festgelegten geometrischen Formen, wie eine Höckerspitze, eine Inzisalkante oder eine Labialfläche, aufweisen, wobei anhand einer Oberfläche mindestens eines Restzahns des jeweiligen Zahns für die einzusetzende Restauration und/oder der Nachbarzähne relativ zu dem jeweiligen Zahn die Zahnnummer und/oder eine Position des Zahns bestimmt werden.

Diese Ausführungsform stellt eine Alternative zum vollautomatischen Maschine-Learning-System aus mindestens einem CNN-Netzwerk dar. Die geometrischen Formen bzw. Merkmale des 3D-Modells der Zahnsituation werden also manuell durch einen Benutzer festgelegt und parametrisiert. Beim Template-matching-Verfahren wird also das zu analysierende 3D-Modell der Zahnsituation auf diese festgelegten geometrischen Formen hin durchsucht. Ein Suchalgorithmus nach einer Höckerspitze könnte beispielsweise auf einem Gradientenverfahren basieren. Der Suchalgorithmus kann also markante geometrische Formen, wie Höckerspitzen, die Inzisalkante oder die Labialfläche erkennen und segmentieren. Auf diese Weise wird die Zahnnummer bestimmt. Denn Eckzähne sind beispielsweise anhand der markanten Höckerspitzen erkennbar.

Vorteilhafterweise kann der ermittelte Restaurationstyp und/oder Zahnnummer mittels einer Anzeigevorrichtung einem Benutzer angezeigt werden.

Das aufgenommene 3D-Modell der Zahnsituation kann also mittels einer Anzeigevorrichtung, wie eines Monitors, angezeigt werden, wobei die ermittelten Informationen, wie der Restaurationstyp und/oder die Zahnnummer, eingeblendet werden können. Die Restauration kann auch konstruiert werden und graphisch innerhalb des vermessenen 3D-Modells der Zahnsituation angezeigt werden.

Vorteilhafterweise kann der ermittelte Restaurationstyp und/oder Zahnnummer verwendet werden, um die Restauration zu konstruieren.

Dadurch wird unter Verwendung des vermessenen 3D-Modells der Zahnsituation, des ermittelten Restaurationstyps und der Zahnnummer die Restauration vollautomatisch konstruiert, wobei beispielsweise markante Strukturen, wie die Präparationskante, die Form der Präparation, die Form der Nachbarzähne und die Form der Gegenzähne, berücksichtigt werden.

Dadurch wird also eine vollautomatische Konstruktion der Restauration ohne eine Benutzerinteraktion ermöglicht.

Vorteilhafterweise kann eine Farbinformation des Restzahns des jeweiligen Zahns für die einzusetzende Restauration und/oder der Nachbarzähne verwendet werden, um eine Farbe für die einzusetzende Restauration festzulegen.

Dadurch kann die Farbe der einzusetzenden Restauration automatisch mittels des Computers ohne jegliche Benutzerinteraktion festgelegt werden. Dadurch wird die Konstruktionsdauer einer Restauration verkürzt. Dadurch werden auch Bedienungsfehler verhindert, die bei einer manuellen Auswahl der Farbe zu einer fehlerhaften Restauration führen könnten.

Vorteilhafterweise kann der ermittelte Restaurationstyp und/oder Zahnnummer verwendet werden, um ein Material für die herzustellende Restauration festzulegen.

Dadurch wird auch das Material für die herzustellende Restauration automatisch mittels eines Computers festgelegt.

Falls die Restauration mittels eines CAD/CAM-Herstellungsverfahrens hergestellt wird, kann automatisch ein passender Rohling aus einem passenden Material ausgewählt werden. Dadurch wird also die Dauer der Herstellung eine Restauration verkürzt.

### Kurzbeschreibung der Zeichnungen

Die Erfindung wird anhand der Zeichnungen erläutert. Es zeigt, die
- Fig. 1: eine Skizze zur Verdeutlichung des Verfahrens zur Konstruktion einer Restauration, die
- Fig. 2: eine Skizze zur Erläuterung der Höhenfelder in okklusaler Richtung, die
- Fig. 3: eine Skizze zur Erläuterung der Höhenfelder in labialer und mesialer Richtung.

### Ausführungsbeispiele

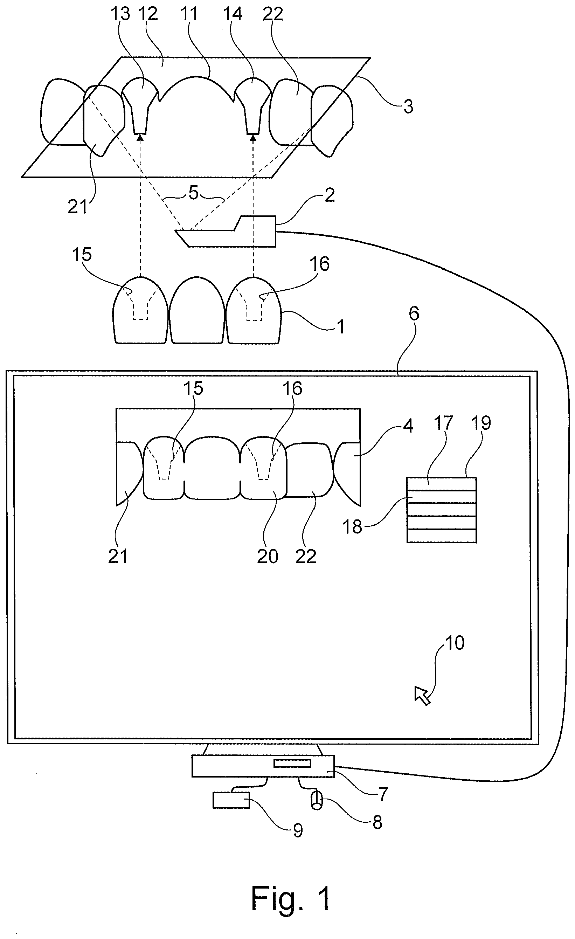

Die Fig. 1 zeigt eine Skizze zur Verdeutlichung des Verfahrens zur Konstruktion einer Restauration 1, wie einer Brücke, wobei mittels einer dentalen Kamera 2 eine Zahnsituation 3 vermessen wird und ein 3D-Modell 4 der Zahnsituation 3 erzeugt wird. Die Vermessung der Zahnsituation 3, die durch ein Rechteck angedeutet ist, mittels der dentalen Kamera 2, ist durch die gestrichelten Linien 5 angedeutet. Das 3D-Modell 4 der Zahnsituation 3 wird mittels einer Anzeigevorrichtung 6, wie einem Monitor, angezeigt, die an einen Computer 7 angeschlossen ist. Die Bilddaten der dreidimensionalen Kamera 2 werden an den Computer 7 weitergeleitet. An den Computer 7 sind Eingabemittel, wie eine Maus 8 und eine Tastatur 9, angeschlossen, so dass ein Benutzer mittels eines Cursors 10 innerhalb der graphischen Darstellung des 3D-Modells 4 navigieren kann. Die Zahnsituation 3 weist einen fehlenden Schneidezahn 11 des Oberkiefers 12 mit einer Zahnnummer 11 nach dem Zahnschema auf. Daneben ist eine erste Präparation 13 des benachbarten Zahns mit der Zahnnummer 12 nach dem Zahnschema angeordnet. Auf der rechten Seite ist eine zweite Präparation 14 in Form eines Zahnstumpfes des benachbarten Zahns mit der Zahnnummer 21 angeordnet. Die zu konstruierende und herzustellende Restauration 1 ist so geformt, dass eine erste Vertiefung 15 zu der ersten Präparation 13 und eine zweite Vertiefung 16 zu der zweiten Präparation 14 passend gefertigt ist. Die Restauration 1 wird also auf die beiden Präparationen 13 und 14 aufgesetzt und verklebt. Mittels eines computergestützten Erkennungsalgorithmus wird das 3D-Modell 4 der Zahnsituation 3 analysiert und ein Restaurationstyp 17 und eine Zahnnummer 18 automatisch ermittelt, wobei der Restaurationstyp 17 und die Zahnnummer 18 in einem Menü 19 mittels der Anzeigevorrichtung 6 angezeigt werden. Anhand des vermessenen 3D-Modells 4 der Zahnsituation 3, des ermittelten Restaurationstyps 17 und ermittelten Zahnnummer bzw. Position der einzusetzenden Restauration 18 wird automatisch ein 3D-Modell 20 der herzustellenden Restauration 1 erzeugt, wobei wesentliche Strukturen, wie die Form der Präparation 15 und der Präparation 16, die Form eines ersten Nachbarzahns 21 und eines zweiten Nachbarzahns 22 berücksichtigt werden. Der erste Nachbarzahn 21 ist dabei ein Eckzahn mit der Zahnnummer 13 und der zweite Nachbarzahn 22 ist ein Zahn mit der Zahnnummer 22 nach dem Zahnschema. Unter Verwendung des konstruierten 3D-Modells 20 kann die Restauration 1 vollautomatisch mittels einer CAM-Bearbeitungsmaschine aus einem Rohling herausgearbeitet werden. Der Vorteil des erläuterten Verfahrens besteht also darin, dass die Restauration 1 nach der Vermessung mittels der dentalen Kamera 2 vollautomatisch hergestellt werden kann, ohne dass eine Benutzerinteraktion erforderlich ist.

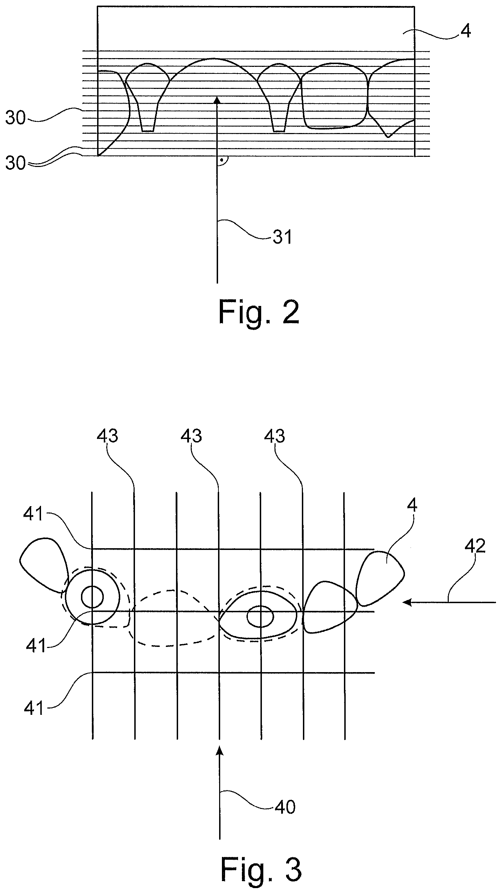

Die Fig. 2 zeigt eine Skizze zur Erläuterung der Höhenfelder 30, die als Eingabedaten für ein CNN-Netzwerk dienen, wobei das CNN-Netzwerk ein Computeralgorithmus ist, der auf dem Computer 7 aus Fig. 1 läuft. Das 3D-Modell 4 wird dabei senkrecht zu einer okklusalen Richtung 31 in gleichen Abständen geschnitten, so dass Schnittbilder bzw. Höhenfelder 30 erzeugt werden. Beim Trainieren eines CNN-Netzwerks wird eine Vielzahl von unterschiedlichen 3D-Modellen unterschiedlicher Zahnsituationen analysiert.

In Fig. 3 wird das 3D-Modell 4 senkrecht zu einer labialen Richtung 40 geschnitten, so dass Höhenfelder 41 bzw. Schnittbilder senkrecht zur labialen Richtung 40 entstehen. Das 3D-Modell 4 wird auch senkrecht zu einer mesialen Richtung 42 geschnitten, so dass Höhenbilder 43 senkrecht zur mesialen Richtung 42 erzeugt werden. Die Schnittbilder bzw. Höhenbilder 30, 41 und 43 dienen als Eingabedaten für das CNN-Netzwerk, wobei als Ausgabedaten des CNN-Netzwerks beispielsweise der Restaurationstyp 17 und die Zahnnummer 18 der einzusetzenden Restauration 1 aus Fig. 1 ermittelt werden.

### Bezugszeichen

- 1: Restauration
- 2: Kamera
- 3: Zahnsituation
- 4: 3D-Modell
- 5: Linien des Aufnahmebereichs
- 6: Anzeigevorrichtung
- 7: Computer
- 8: Maus
- 9: Tastatur
- 10: Cursor
- 11: Schneidezahn
- 12: Oberkiefer
- 13: Präparation
- 14: zweite Präparation
- 15: Vertiefung
- 16: zweite Vertiefung
- 17: Restaurationstyp
- 18: Zahnnummer der einzusetzenden Restauration
- 19: Menü
- 20: 3D-Modell
- 21: Nachbarzahn
- 22: zweiter Nachbarzahn
- 30: Höhenfelder
- 31: okklusale Richtung
- 40: labiale Richtung
- 41: Höhenfelder
- 42: mesiale Richtung
- 43: Höhenbilder

## Patentansprüche

1. Verfahren zur Konstruktion einer Restauration (1), wobei mittels einer dentalen Kamera (2) eine Zahnsituation (3) vermessen wird und ein 3D-Modell (4) der Zahnsituation (3) erzeugt wird, **dadurch gekennzeichnet, dass** ein computergestützter Erkennungsalgorithmus auf das 3D-Modell (4) der Zahnsituation (3) angewendet wird, wobei ein Restaurationstyp (17) der einzusetzenden Restauration (1) automatisch ermittelt wird, wobei die Zahnsituation (3) mindestens eine Präparation (13, 14) oder eine implantatgetragene Mesostruktur zum Einsetzen der herzustellenden Restauration (1) aufweist, wobei der computergestützte Erkennungsalgorithmus ein künstliches neuronales Netz für maschinelles Lernen aufweist, wobei anhand des 3D-Modells (4) der Zahnsituation (3) die Form der Präparation (13, 14) oder der implantatgetragenen Mesostruktur mittels eines Maschinen-Learning-Systems analysiert wird und ein passender Restaurationstyp (17) ausgewählt wird, wobei das Maschinen-Learning-System aus einem oder mehreren CNN Netzwerken besteht.

2. Verfahren nach Anspruch 1, **dadurch gekennzeichnet, dass** der Restaurationstyp (17) ein Inlay, eine Krone, eine Brücke, ein Abutment, ein Pontic oder ein Veneer ist.

3. Verfahren nach einem der Ansprüche 1 oder 2, **dadurch gekennzeichnet, dass** der ermittelte Restaurationstyp (17) mittels einer Anzeigevorrichtung (6) einem Benutzer angezeigt wird.

4. Verfahren nach einem der Ansprüche 1-3, **dadurch gekennzeichnet, dass** der ermittelte Restaurationstyp (17) verwendet wird, um die Restauration (1) zu konstruieren.

5. Verfahren nach einem der Ansprüche 1-4, **dadurch gekennzeichnet, dass** eine Farbinformation des Restzahns des jeweiligen Zahns für die einzusetzende Restauration (1) und/oder der Nachbarzähne (21, 22) verwendet wird, um eine Farbe für die einzusetzende Restauration festzulegen.

6. Verfahren nach einem der Ansprüche 1-5, **dadurch gekennzeichnet, dass** der ermittelte Restaurationstyp (17) verwendet wird, um ein Material für die herzustellende Restauration festzulegen.

## Claims

1. A method for constructing a restoration (1), wherein a dental situation (3) is measured by means of a dental camera (2) and a 3D model (4) of the dental situation (3) is generated, **characterised in that** a computer-aided recognition algorithm is applied to the 3D model (4) of the dental situation (3), wherein a restoration type (17) of the restoration (1) to be inserted is determined automatically, wherein the dental situation (3) has at least one preparation (13, 14) or an implant-supported mesostructure for inserting the restoration (1) to be produced, wherein the computer-aided recognition algorithm has an artificial neural network for machine learning, wherein, on the basis of the 3D model (4) of the dental situation (3), the shape of the preparation (13, 14) or of the implant-supported mesostructure is analysed by means of a machine learning system and a suitable restoration type (17) is selected, wherein the machine learning system consists of one or more CNN networks.

2. The method according to claim 1, **characterised in that** the restoration type (17) is an inlay, a crown, a bridge, an abutment, a pontic or a veneer.

3. The method according to any one of claims 1 or 2, **characterised in that** the determined restoration type (17) is displayed to a user by means of a display device (6).

4. The method according to any one of claims 1-3, **characterised in that** the determined restoration type (17) is used to construct the restoration (1).

5. The method according to any one of claims 1-4, **characterised in that** colour information of the residual tooth of the relevant tooth for the restoration (1) to be inserted and/or of the neighbouring teeth (21, 22) is used in order to define a colour for the restoration to be inserted.

6. The method according to any one of claims 1-5, **characterised in that** the determined restoration type (17) is used to define a material for the restoration to be produced.

## Revendications

1. Procédé de réalisation d'une restauration (1), une caméra dentaire (2) étant utilisée pour mesurer une situation dentaire (3) et un modèle 3D (4) de la situation dentaire (3) étant généré, **caractérisé en ce qu'**un algorithme de reconnaissance assistée par ordinateur est utilisé sur le modèle 3D (4) de la situation dentaire (3), un type de restauration (17) de la restauration (1) à insérer étant déterminé automatiquement, la situation dentaire (3) présentant au moins une préparation (13, 14) ou une mésostructure implanto-portée pour l'insertion de la restauration (1) à réaliser, l'algorithme de reconnaissance assistée par ordinateur présentant un réseau neuronal artificiel pour l'apprentissage automatique, dans lequel, à l'aide du modèle 3D (4) de la situation dentaire (3) on analyse la forme de la préparation (13, 14) ou de la mésostructure implanto-portée au moyen d'un système d'apprentissage automatique et on sélectionne un type de restauration approprié (17), le système d'apprentissage automatique étant constitué d'un ou plusieurs réseaux CNN.

2. Procédé selon la revendication 1, **caractérisé en ce que** le type de restauration (17) est un inlay, une couronne, un bridge, un pilier, un pontique ou une facette.

3. Procédé selon l'une quelconque des revendications 1 ou 2, **caractérisé en ce que** le type de restauration (17) déterminé est affiché à un utilisateur au moyen d'un dispositif d'affichage (6).

4. Procédé selon l'une quelconque des revendications 1-3, **caractérisé en ce que** le type de restauration (17) déterminé est utilisé pour réaliser la restauration (1).

5. Procédé selon l'une quelconque des revendications 1-4, **caractérisé en ce qu'**on utilise des informations de couleur de la dent restante de la dent considérée pour la restauration (1) à insérer et/ou des dents voisines (21, 22) afin de définir une couleur pour la restauration à insérer.

6. Procédé selon l'une quelconque des revendications 1-5, **caractérisé en ce que** le type de restauration (17) déterminé est utilisé pour définir un matériau pour la restauration à réaliser.
